# NEUE EUROPÄISCHE PATENTSCHRIFT

(11) **EP 1 127 566 B2**
(45) Veröffentlichungstag und Bekanntmachung des Hinweises auf die Entscheidung über den Einspruch: **05.03.2008**
(45) Hinweis auf die Patenterteilung: 08.06.2005
(21) Anmeldenummer: 01100617.8
(22) Anmeldetag: 11.01.2001
(51) Int. Cl.: A61Q 5/06, A61K 8/41, A61K 8/49, A61K 8/81

(54) **Haarfärbemittel**
Hair dyeing composition
Compositon de coloration capillaire

(30) Priorität: 21.02.2000 DE 10007776
(43) Veröffentlichungstag der Anmeldung: 29.08.2001
(73) Patentinhaber: KPSS-Kao Professional Salon Services GmbH, 64297 Darmstadt (DE)
(72) Erfinder: Lorenz, Heribert, 64401 Gross-Bieberau (DE)

(56) Entgegenhaltungen:
- EP-A- 0 257 807
- EP-A- 0 953 334
- EP-A- 0 960 617
- EP-A- 1 004 288
- DE-A- 3 423 349
- DE-A- 3 423 589
- US-A- 5 679 114

## Beschreibung

Die Erfindung betrifft ein Haarfärbemittel auf wäßriger Basis, das mindestens einen direktziehenden Haarfarbstoff enthält.
Derartige Haarfärbemittel sind seit langem bekannt.
Sie enthalten meistens mehrere anionische oder kationische direktziehende Farbstoffe und bedürfen, im Gegensatz zu den permanenten Haarfärbemitteln auf Basis von Oxidationsfarbstoffvorprodukten, keiner vorherigen Entwicklung mit Oxidationsmitteln.

Diese direktfärbenden Zusammensetzungen werden entweder zusammen mit oberflächenaktiven Mitteln als sogenannte Tönungsshampoos appliziert, oder, wie die erfindungsgemäßen Zusammensetzungen, als Lotionen, Emulsionen oder verdickte Lösungen, d.h., Gele, auf das Haar aufgebracht.

Sie müssen einerseits so viskos sein, daß sie auf dem Haar verbleiben, um eine ausreichende Färbewirkung zu gewährleisten, müssen sich aber andererseits nach vollendeter Färbung wieder vollständig und leicht aus dem Haar ausspülen lassen. Derartige Zusammensetzungen enthalten also in der Regel ein Verdickungsmittel. Die Zahl der hierfür vorgeschlagenen Substanzen ist hoch, eine optimale Wirksamkeit und Verträglichkeit mit den eingesetzten direktziehenden Farbstoffen vermögen sie nicht auszuüben.
Das gilt auch für die in der DE 34 23 349 C2 beschriebenen Färbemittel, die Xanthan-Gummi als Verdickungsmittel enthalten: ihr Einsatz ist vorwiegend auf Nitrobenzolderivate als Farbstoffe aufweisende Mittel beschränkt.

DE 342 35 89 beschreibt Haarfärbemittel die Oxidationsfarbstoffe und eine quatemisiertes Dimethylaminoethylmethacrylat - Homopolymerisat enthalten. Haarfärbemittel die als Farbstoff hauptsächlich direktziehende Farbstoffe enthalten sind nicht beschrieben.

Die Erfindung geht daher von der Aufgabenstellung aus, ein Haarfärbemittel auf wäßriger Basis zu schaffen, das als Lotion, Emulsion, Lösung, Gel, Suspension bzw. auch, unter Zusatz von Treibmitteln, als Aerosolzusammensetzung auf das Haar aufgebracht werden kann, das optimale rheologische Eigenschaften aufweist, über längere Zeit stabil bleibt, und sowohl mit anionischen als auch kationischen direktziehenden Haarfarbstoffen ausgezeichnete färberische Eigenschaften zeigt.

Die Lösung dieser Aufgabe besteht darin, einem Mittel, das einen pH-Wert zwischen 2 und 7 aufweist, ein Polydimethyloder ein Polydiethylamino-C₂-C₃-alkylmethacrylat-Polymerisat, das vorzugsweise quaternisiert sein kann, und mindestens ein kationisches Tensid zuzusetzen.

Dieses Polymer ist sowohl mit kationischen als auch mit anionischen Farbstoffen ausgezeichnet kompatibel; die Zusammensetzungen sind über längere Zeit selbst bei sauren pH-Werten lagerstabil und ergeben intensive, glänzende Haarfärbungen. Zusätzlich wird auch noch eine haarkonditionierende Wirkung, insbesondere eine verbesserte Naß- und Trockenkämmbarkeit, lockerer Griff, Volumen und Spannkraft erzielt; jedwede beim wiederholten Färben des Haares zuweilen auftretende Rauhigkeit des Haares wird vermieden.

Darüber hinaus sind die mit den erfindungsgemäßen Haarfärbemitteln erzielten Färbungen auch äußerst stabil und überstehen nahezu unverändert auch mehrere Haarwäschen.

Derartige Polydimethylaminoethylmethacrylate bzw. Polydiethylaminoethyl- bzw. -aminopropylmethacrylate werden durch Polymerisation der entsprechenden Monomeren gewonnen und sind auch, beispielsweise unter der Bezeichnung Polyquaternium 37", auf dem Markt erhältlich, z. B. als 50%-ige Dispersion in organischem Medium, z.B. flüssigen Fettsäureestern und/oder Fettalkoholen.

Besonders bevorzugt ist die Verwendung eines mit Methylchlorid oder Dimethyloder Diethylsulfat quaternierten Dimethylaminoethylmethacrylat-Homopolymerisats.

Der Anteil des Dimethyl- bzw. Polydiethylaminoethyl- bzw. propylmethacrylat-Polymeren in den erfindungsgemäßen Haarfärbemitteln liegt bei etwa 0,25 bis etwa 10, vorzugsweise etwa 0,5 bis etwa 7,5, insbesondere etwa 1 bis etwa 5 Gew.-%, berechnet auf die Gesamtzusammensetzung des Mittels.

Es ist im Prinzip möglich jedoch nicht erforderlich, der erfindungsgemäßen Zusammensetzung weitere bekannte Verdickungsmittel zuzusetzen, soweit sie mit deren weiteren Bestandteilen, insbesondere den kationischen oder anionischen Farbstoffen bei sauren pH-Werten, verträglich sind.
Solche Verdickungsmittel sind dem Fachmann an sich bekannt und bedürfen deshalb keiner Aufzählung.

Der Anteil der direktziehenden Farbstoffe in den erfindungsgemäßen Zusammensetzungen ist variabel und liegt zwischen etwa 0,005 bis etwa 5, vorzugsweise 0,01 bis 2,5, insbesondere 0,1 bis 1 Gew.-% des Mittels.

Als direktziehende Haarfarbstoffe können im Prinzip alle für diesen Zweck vorgeschlagenen kationischen Farbstoffe verwendet werden.

Bevorzugt sind die sogenannten "Arianor"-Farbstoffe; vgl. K. Schrader, "Grundlagen und Rezepturen der Kosmetika", 2. Aufl. (1989), S. 811.

Besonders geeignete basische (kationische) Farbstoffe sind:

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16, | C.I.-No. 12,250; |
| Basic Brown 17, | C.I.-No. 12,251; |
| Natural Brown 7, | C.I.-No. 75,500; |
| Basic Green 1, | C.I.-No. 42,040; |
| Basic Red 2, | C.I.-No. 50,240; |
| Basic Red 22, | C.I.-No. 11,055; |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, | C.I.-No. 42,510; |
| Basic Yellow 57, | C.I.-No. 12,719. |

Selbstverständlich ist auch die Verwendung entsprechender direktziehender Pflanzenfarbstoffe oder auch anionischer (saurer) direktziehender Haarfarbstoffe möglich.

Diese werden üblicherweise ebenfalls in einer Menge von etwa 0,005 bis etwa 5, vorzugsweise etwa 0,05 bis etwa 2,5, insbesondere etwa 0,1 bis etwa 1 Gew.-%, berechnet auf die Gesamtzusammensetzung des Mittels,das als Lösung, Dispersion, Emulsion, Gel oder Aerosolpräparat zur direkten Anwendung vorliegt, eingesetzt.

Als geeignete anionische Farbstoffe können beispielsweise Verwendung finden:

| | |
|---|---|
| Acid Black 1, | C.I.-No. 20,470; |
| Acid Blue 1, | C.I.-No. 42,045; |
| Food Blue 5, | C.I.-No. 42,051; |
| Acid Blue 9, | C.I.-No. 42,090; |
| Acid Blue 74, | C.I.-No. 73,015; |
| Acid Red 18, | C.I.-No. 16,255; |
| Acid Red 27, | C.I.-No. 16,185; |
| Acid Red 87, | C.I.-No. 45,380; |
| Acid Red 92, | C.I.-No. 45,410; |
| Acid Orange 7, | C.I.-No. 15,510; |
| Acid Violet 43, | C.I.-No. 60,730; |
| Acid Yellow 1, | C.I.-No. 10,316; |
| Acid Yellow 23, | C.I.-No. 19,140; |
| Acid Yellow 3, | C.I.-No. 47,005; |
| Food Yellow No. 8, | C.I.-No. 14,270; |
| D&C Brown No. 1, | C.I.-No. 20,170 |
| D&C Green No. 5, | C.I.-No. 61,570; |
| D&C Orange No. 4, | C.I.-No. 15,510; |
| D&C Orange No. 10, | C.i.-No 45,425:1; |
| D&C Orange No. 11, | C.I.-No. 45,425; |
| D&C Red No. 21, | C.I.-No. 45,380:2; |
| D&C Red No. 27, | C.I.-No. 45,410:1; |
| D&C Red No. 33, | C.I.-No. 17,200; |
| D&C Yellow No. 7, | C.I.-No. 45,350:1; |
| D&C Yellow No. 8, | C.I.-No. 45,350; |
| FD&C Red No. 4, | C.I.-No. 14,700; |
| FD&C Yellow No. 6, | C.I.-No. 15,985. |

Auch pflanzliche Farbstoffe können allein oder in Kombination mit synthetischen Direktziehern Verwendung finden, beispielsweise Henna (rot oder schwarz), Alkannawurzel, Laccainsäure (Stocklack), Indigo, Blauholzpulver, Krappwurzel- und Rhabarberwurzelpulver, etc.

Das erfindungsgemäße Haarfärbemittel kann auch oberflächenaktive Substanzen enthalten, obwohl es sich um kein Tönungsshampoo handelt.

Solche können anionisch, nichtionisch, kationisch oder amphoter bzw. zwitterionisch sein.

Bevorzugt sind nichtionische und kationische Tenside in einer Menge zwischen etwa 0,5 und etwa 5 Gew.-%, berechnet auf die Gesamtzusammensetzung.

Geeignete nichtionische Tenside sind Verbindungen aus der Klasse der Alkylpolyglucoside mit der allgemeinen Formel

R-O-(CH₂CH₂O)ₙ-Zₓ,

worin R eine Alkylgruppe mit 8 bis 20, vorzugsweise 10 bis 14 Kohlenstoffatomen, Zₓ einen Saccharidrest mit 5 bis 6 Kohlenstoffatomen, n, eine Zahl von 0 bis 10, und x eine Zahl zwischen 1 und 5, vorzugsweise 1,1 bis 2,5 bedeuten.

Weitere geeignete nichtionische Tenside in den erfindungsgemäßen Mitteln sind C₁₀-C₂₂-Fettalkoholethoxylate.

Besonders geeignete C₁₀-C₂₂-Fettalkoholether sind die unter den Trivialnamen "Laureth", "Myristeth", "Oleth", Ceteth", "Deceth", "Steareth" und "Ceteareth" nach der CTFA-Nomenklatur mit Anfügung der Zahl der Ethylenoxid-Moleküle bezeichneten Alkylpolyglykolether, z.B. "Laureth-16":

Der durchschnittliche Ethoxylierungsgrad liegt dabei zwischen etwa 2,5 und etwa 25, vorzugsweise etwa 10 und etwa 20.

Andere zusätzlich mitverwendbare nichtionische Tenside sind z.B. die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics®" im Verkehr sind.

Weitere zusätzlich einsetzbare Tenside sind Aminoxide.
Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyloder ethylaminoxide, C₁₂-C₁₈-Alkyldi(hydroxyethyl)-oder -(hydroxypropyl)aminoxide, oder auch Aminoxide mit Ethylenoxid- und/oder Propylenoxidgruppen in der Alkylkette.

Geeignete Aminoxide sind beispielsweise unter den Bezeichnungen "Ammonyx®", "Aromox®", oder "Genaminox®" im Handel.

Weitere fakultative Tensidbestandteile sind Fettsäuremono- und dialkanolamide, wie Cocosfettsäuremonoethanolamid und Myristinfettsäuremonoisopropanolamid.

Geeignete amphotere bzw. zwitterionische Tenside sind insbesondere die verschiedenen bekannten Betaine wie Fettsäureamidoalkylbetaine und Sulfobetaine, beispielsweise Laurylhydroxysulfobetain; auch langkettige Alkylaminosäuren wie Cocoaminoacetat, Cocoaminopropionat und Natriumcocoamphopropionat und acetat haben sich als geeignet erwiesen.

In einzelnen können Betaine der Struktur 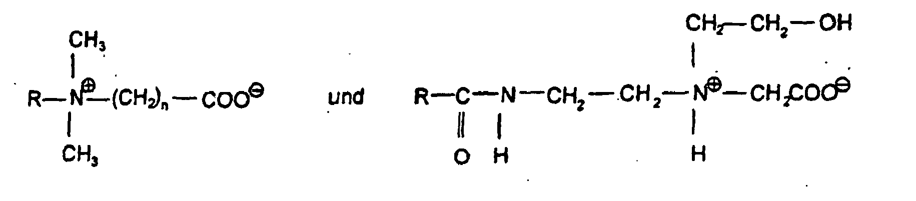 wobei R eine C₈-C₁₈-Alkylgruppe und n 1 bis 3 bedeuten,

Sulfobetaine der Struktur 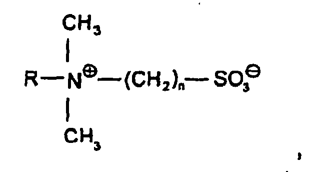 wobei R eine C₈-C₁₈-Alkylgruppe und n 1 bis 3 bedeuten,
und Amidoalkylbetaine der Struktur 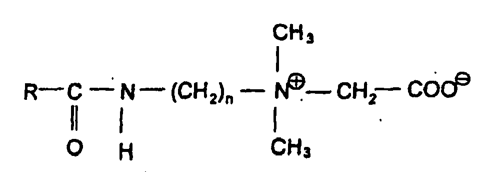 wobei R eine C₈-C₁₈-Alkylgruppe und n 1 bis 3 bedeuten, verwendet werden. Bevorzugt sind Fettsäureamidoalkylbetaine, insbesondere Cocoamidopropylbetain, und Cocoamphoacetat und-propionat, insbesondere deren Natriumsalze.
Besonders bevorzugt sind Gemische aus Cocoamidopropylbetain und Cocoamphoacetat, insbesondere im Gewichtsverhältnis 3:1 bis 1:3, vor allem 2:1 bis 1:1.

Geeignete kationische Tenside sind beispielsweise langkettige quaternäre Ammoniumverbindungen, die allein oder im Gemisch miteinander eingesetzt werden können, wie Cetyltrimethylammoniumchlorid, Dimethyldistearylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxy-ethyl)alkylammoniumphosphat, Cetylpyridiniumchlorid, etc. Gut geeignet sind auch die in der EP-A 472 107 geoffenbarten quaternären Ammoniumsalze.

Weitere geeignete langkettige Ammoniumverbindungen sind Esterquats der allgemeinen Formel (I) 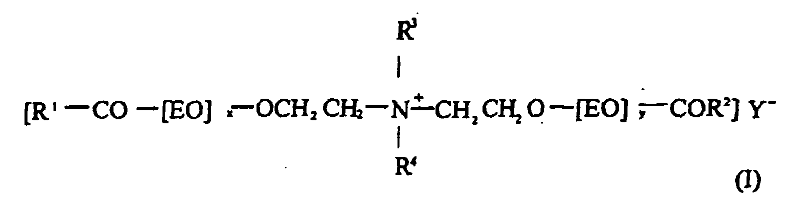 in der R¹ und R² für eine gegebenenfalls hydroxysubstituierte C₈-C₂₂-Alkyloder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y⁻ für ein Anion stehen.

Eine besonders bevorzugte Verbindung der Formel 1 ist im Rahmen der Erfindung eine solche, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[EO]_{z}-H bedeuten.

Das Anion Y⁻ ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.

Diese Verbindungen sind an sich bekannt und beispielsweise unter den Handelsnamen "Schercoquat^{R}", "Dehyquart^{R}F30" und "Tetranyl^{R}" im Handel.

Der Einsatz dieser "Esterquats" in Haarpflegemitteln ist an sich bekannt und beispielsweise in der WO-A 93/10748, der WO-A 92/06899 und der WO-A 94/16677 beschrieben.

Geeignet sind auch Amidoquats der allgemeinen Formel (II)

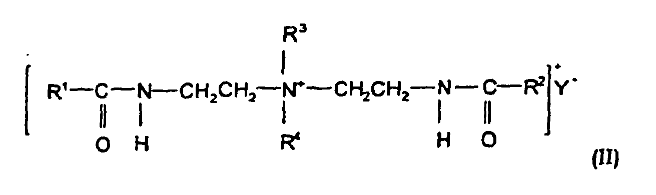

in der R¹ und R² jeweils für eine gegebenenfalls hydroxysubstituierte C₈-C₂₂-Alkyloder Alkenyigruppe, R³ und R⁴ für eine C₁-C₃-Alkylgruppe oder eine Gruppe-CH₂-CH₂-O-[EO]ₓ-H, sowie x für 0 bis 5, Y⁻ für ein Anion stehen.

Es können selbstverständlich auch Gemische aus den verschiedenen Tensiden, soweit sie miteinander kompatibel sind, verwendet werden.

Ein weiterer wünschenswerter Bestandteil der erfindungsgemäßen Färbemittel-Zusammensetzungen ist ein C₃-C₆-Alkandiol bzw. dessen Ether, insbesondere ein Mono-C₁-C₃-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen 0,5 und 30, vorzugsweise etwa 1 bis etwa 15, insbesondere etwa 5 bis etwa 10 Gew.-% der Färbemittel-Zusammensetzung.

Neben den C₃-C₆-Alkandiolen bzw. deren Ethern können zusätzlich auch Monoalkohole wie Ethanol, Propanol-1, Propanol-2 sowie Polyalkohole wie Glycerin und Hexantriol, Ethylcarbitol, Benzylalkohol, Benzyloxyethanol sowie Propylencarbonat (4-Methyl-1,3-dioxolan-2-on), N-Alkylpyrrolidone und Harnstoff Verwendung finden.

Weitere mögliche zusätzliche Bestandteile sind kationische, anionische, nichtionische und amphotere Polymere, vorzugsweise in einer Menge von etwa 0,1 bis etwa 5, insbesondere etwa 0,25 bis 2,5 Gew.-% der Gesamtzusammensetzung des Färbemittels.

Diese Mittel können auch weitere Pflegemittel wie Öle und Fette enthalten. Solche sind beispielsweise Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweise Silikonöle, Polyethylenglykole, etc.

Weitere geeignete hydrophobe Komponenten sind insbesondere Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.
Falls es sich bei den erfindungsgemäßen Mitteln um Emulsionen handelt, enthalten diese selbstverständlich die üblichen Emulgatoren.

Die erfindungsgemäßen Mittel können auch langkettige Fettsäuren enthalten.
Als Fettsäuren werden bevorzugt solche mit 10 bis 24, insbesondere 12 bis 22 Kohlenstoffatomen in einer Menge von etwa 0,5 bis 15 Gew.-%, insbesondere 1 bis 10 Gew.-%, bezogen auf die Gesamtzusammensetzung, verwendet. Besonders geeignet sind Behensäure und Stearinsäure; jedoch können auch andere Fettsäuren wie beispielsweise Myristinsäure, Palmitinsäure oder Ölsäure oder auch Gemische natürlicher oder synthetischer Fettsäuren wie Kokosfettsäure eingesetzt werden.

Die Viskosität der erfindungsgemäßen Mittel liegt vorzugsweise bei etwa 5000 bis etwa 60 000, insbesondere etwa 10 000 bis 50 000, vor allem etwa 20 000 bis 40 000 mPa.s bei 20°C, gemessen im Brookfield-Rotationsviskosimeter mit einer Spindel Nr. 5 bei 5rpm.

Der pH-Wert liegt im sauren Bereich zwischen 2 und 7, vorzugsweise 2,5 und 6, insbesondere etwa 3 und 5,5.

Das folgende Beispiel dient der Illustration der Erfindung.

### Beispiel 1

| | |
|---|---|
| Polyquaternium-6 | 0,60 (Gew.-%) |
| Cetrimoniumchlorid | 0,80 |
| 1,2-Propandiol | 0,40 |
| Phenoxypropanol | 0,10 |
| Parfum | 0,30 |
| Quaternäres Polydimethylaminoethylaminoethylmethacrylat-Homopolymerisat (Polyquaternium-37; 50%-ig) | 6,00 |
| Basic Blue 99 (C.I.-No 56059) | 0,04 |
| Basic Brown 16 (C.I.-No. 12250) | 0,36 |
| HC-Red 3 | 0,17 |
| HC-Yellow 5 | 0,33 |
| Wasser | ad 100,00 |
| pH-Wert: | 6,5 |
| Viskosität bei 20°C im Rotationsviskosimeter nach Brookfield (Spindel Nr. 5, 5rpm): | ~12 000 mPa.s |

Die Zusammensetzung blieb bei viermonatiger Lagerung bei 40°C völlig stabil.

Bei der Haarfärbung wurde ein glänzender Kupferrotton erhalten, der nach fünf Haarwäschen noch deutlich war.

Es wurde eine zusätzliche deutliche haarkonditionierende Wirksamkeit erzielt.

## Patentansprüche

1. Haarfärbemittel auf wäßriger Basis das einen pH-Wert zwischen 2 und 7 aufweist, enthaltend
a) mindestens einen direktziehenden Haarfarbstoff;
b) mindestens ein gegebenenfalls quatemisiertes Dimethyl- oder Diethylamino-C₂-C₃-alkylmethacrylat-Polymerisat, und
c) mindestens ein kationisches Tensid.

2. Haarfärbemittel nach Anspruch 1, enthaltend 0,25 bis 10 Gew.-% des Dimethyloder Diethylamino-C₂-C₃-alkylmethacrylat-Polymers.

3. Haarfärbemittel nach Anspruch 1 oder 2, enthaltend ein quaternäres Polydimethylaminoethylhomopolymerisat.

4. Haarfärbemittel nach einem oder mehreren der Ansprüche 1 bis 3, das einen pH-Wert zwischen 2,5 und 6,0 aufweist.

## Claims

1. Hair dyeing composition on aqueous basis, having a pH-value between 2 and 7, comprising
a) at least one direct-acting dyestuff,
b) at least one optionally quaternized dimethyl or diethyl amino-C₂-C₃-alkyl methacrylate polymer, and
c) at least one cationic surfactant.

2. Hair dyeing composition according to claim 1, containing 0.25 % to 10 % by weight, calculated to the total composition, of the dimethyl- or diethylamino-C₂-C₃-alkyl methacrylate polymer.

3. Hair dyeing composition according to claim 1 or 2, containing a quaternary polydi-methyl aminoethyl homopolymer.

4. Hair dyeing composition according to one or more of claims 1 to 3, having a pH-value between 2.5 and 6.0.

## Revendications

1. Teinture capillaire à base aqueuse, qui présente une valeur de pH comprise entre 2 et 7, contenant
a) au moins un colorant direct des cheveux sur les fibres,
b) au moins un polymère de méthacrylate de diméthyl- ou diéthyl-amino-(alkyle en C₂ à C₃) éventuellement quaternisé, et
c) au moins un tensioactif cationique.

2. Teinture capillaire selon la revendication 1, contenant 0,25 à 10% en poids du polymère de méthacrylate de diméthyl- ou diéthyl-amino-(alkyle en C₂ à C₃).

3. Teinture capillaire selon la revendication 1 ou 2, contenant un homopolymère polydiméthylaminoéthyle quaternisé.

4. Teinture capillaire selon l'une ou plusieurs des revendications 1 à 3, qui présente une valeur de pH comprise entre 2,5 et 6,0.
